# EUROPEAN PATENT APPLICATION

(11) **EP 4 644 372 A1**
(43) Date of publication of application: **05.11.2025**
(21) Application number: 23909632.4
(22) Date of filing: 01.11.2023
(51) Int. Cl.: C07D 213/22, C07D 471/14, A01N 43/40, A01N 43/90, A01P 13/00

(54) **NON-BIPYRIDYL SALT HERBICIDE, PREPARATION METHOD THEREFOR, AND USE THEREOF**

(30) Priority: 26.12.2022 CN 202211674300
(71) Applicant: Shanghai Institute of Organic Chemistry, Chinese Academy of Sciences, Shanghai 200032 (CN); Shanghai Tenth People's Hospital, Shanghai 200072 (CN)
(72) Inventor: TANG, Wenjun, Shanghai 200032 (CN); PENG, Ai, Shanghai 200072 (CN); PENG, Henian, Shanghai 200032 (CN); ZHAO, Dake, Shanghai 200072 (CN)
(74) Representative: Mewburn Ellis LLP
(86) International application number: PCT/CN2023/128950
(87) International publication number: WO 2024/139699

(57) **Abstract**

Disclosed are a non-bipyridyl salt herbicide, a preparation method therefor, and a use thereof. Specifically, the present invention provides a diene diamine compound having the structure of formula (I) or a tricyclic diene piperazine structure represented by formula (II). The non-bipyridyl salt compound can be converted into paraquat or diquat under light and air conditions, has equivalent herbicidal properties to paraquat or diquat, and the toxicity thereof is not significant. Therefore, the non-bipyridyl salt compound of the present invention can be used as an herbicide as a non-toxic substitute for paraquat or diquat.

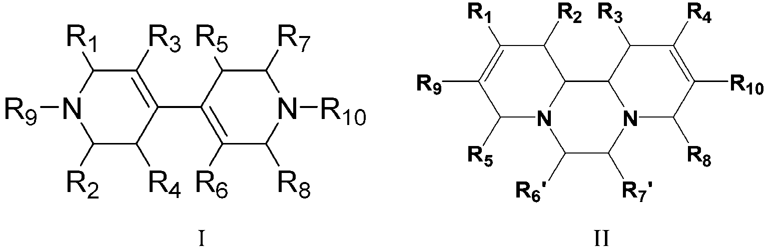

## Description

### TECHNICAL FIELD

The present invention relates to the field of pesticides, specifically, the present invention relates to a diene diamine or tricyclic diene piperazine herbicide, preparation method therefor, and use thereof.

### BACKGROUND

Weeds pose a serious threat to sustainable grain production safety. Herbicides play an irreplaceable role in ensuring global grain production safety. Paraquat (PQ; 1,1-dimethyl-4,4-dipyridinium) dichloride is a non selective herbicide that has been widely used in over 120 countries since the 1960s due to its rapid, efficient, broad-spectrum killing activity against weeds, its ability to maintain root integrity, rapid deactivation upon contact with soil, and relatively low cost as well as other features. Due to the high safety for the environment and high efficiency in weed control, PQ is still an irreplaceable product among herbicides.

However, the high toxicity of PQ to humans has become a serious social issue. The high mortality rate of PQ poisoning is due to its inherent toxicity and lack of effective treatment measures. PQ has caused thousands of deaths worldwide due to suicide and accidental poisoning. Data shows that PQ leads to 2000 times toxic ingestion incidents annually, with a mortality rate of 50-90%. Based on this practical challenge, many countries restrict or prohibit the use of PQ as a herbicide.

Several methods have been reported to reduce the toxicity of PQ to humans and animals and to develop antidotes for PQ. For example, mixing PQ with emetics or using PQ loaded supramolecular vesicles such as pills [6] arenes and curubit [8] uril to develop user safe PQ formulations. Due to its strong ability to complex with PQ, curubit [8] uril is also considered as a potent antidote. Many antioxidants such as naringin, lysine acetylsalicylic acid, selenium, quercetin, vitamin C, and sodium salicylate are used as antioxidant strategies to treat PQ poisoning. However, so far, these intervention strategies have not significantly reduced the mortality rate of PQ poisoning, and the development of non-toxic and effective herbicides as alternatives to PQ still has important practical significance.

In view of this, there is an urgent need in this field for a non-toxic, practical, cost-effective, and high-performance herbicide to replace the highly toxic paraquat.

### SUMMARY OF THE INVENTION

One purpose of the present invention is to provide a non-toxic, practical, cost-effective, and high-performance herbicide as a substitute for paraquat or diquat.

Another purpose of the present invention is to provide a method for preparing a novel herbicide.

Another purpose of the present invention is to provide a method for removing weeds from farmland.

In the first aspect of the present invention, provided is a use of a compound having a structure shown in Formula I or Formula II, pesticide science acceptable salts, enantiomers, diastereomers, optical isomers, tautomers, racemates, deuterated derivatives thereof, or combinations thereof, for the preparation of plant killing agents (preferably herbicides): 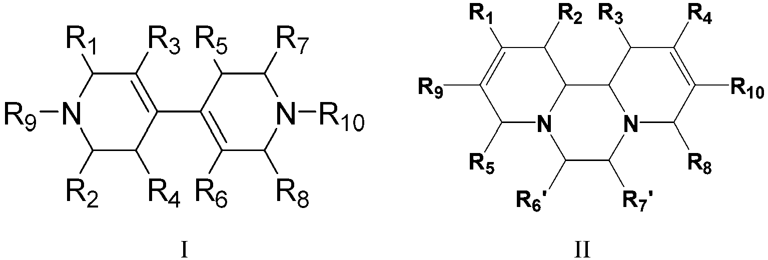 wherein,
R₁, R₂, R₃, R₄, R₅, R₆, R₇, R₈, R₉, and R₁₀ are each independently selected from the group consisting of: hydrogen, halogen, cyano, C₁-C₆ alkyl, C₃-C₆ cycloalkyl, C₂-C₆ alkenyl, C₂-C₆ alkynyl, C₁-C₆ alkoxy, -C(O)O(C₁- C₆)alkyl, -S(O)ₚ(C₁- C₆)alkyl, C₁- C₆ haloalkyl, C₁-C₆ haloalkoxy, and -NRaRb;
p is 0, 1, or 2;
Ra and Rb are each independently selected from the group consisting of: H, C₁-C₆ alkyl, C₃-C₆ cycloalkyl, C₂-C₆ alkenyl, C₂-C₆ alkynyl, C₁-C₆ haloalkyl and C₁-C₆ alkoxy;
R₆' and R₇' are each independently selected from the group consisting of H, C₁-C₆ alkyl, and C₁-C₆ haloalkyl.

In another preferred embodiment, the compound has a structure shown in formula I, and wherein,
R₁, R₂, R₃, R₄, R₅, R₆, R₇, and R₈ are each independently selected from the group consisting of: hydrogen, halogen, cyano, C₁-C₆ alkyl, C₃-C₆ cycloalkyl, C₂-C₆ alkenyl, C₂-C₆ alkynyl, C₁-C₆ alkoxy, -C(O)O(C₁- C₆) alkyl, -S(O)ₚ(C₁- C₆) alkyl, C₁- C₆ haloalkyl, C₁- C₆ haloalkoxy, and -NRaRb;
R₉ and R₁₀ are each independently selected from the group consisting of: C₁-C₆ alkyl, C₃-C₆ cycloalkyl, C₂-C₆ alkenyl, C₂-C₆ alkynyl, and C₁-C₆ haloalkyl;
Ra and Rb are each independently selected from the group consisting of H, C₁-C₆ alkyl, C₃-C₆ cycloalkyl, C₂-C₆ alkenyl, and C₂-C₆ alkynyl.

In another preferred embodiment,
R₁, R₂, R₃, R₄, R₅, R₆, R₇, and R₈ are each independently selected from the group consisting of: hydrogen, halogen, and cyano;
R₉ and R₁₀ are each independently selected from the group consisting of: C₁-C₆ alkyl, and C₃-C₆ cycloalkyl.

In another preferred embodiment, R₁, R₂, R₃, R₄, R₅, R₆, R₇, and R₈ are all H, and R₉ and R₁₀ are C₁-C₄ alkyl.

In another preferred embodiment, the compound has a structure shown in Formula II, and
R₁, R₂, R₃, R₄, R₅, R₈, R₉, and R₁₀ are each independently selected from the group consisting of: hydrogen, halogen, cyano, C₁-C₆ alkyl, C₃-C₆ cycloalkyl, C₂-C₆ alkenyl, and C₂-C₆ alkynyl;
R₆' and R₇' are each independently selected from the group consisting of: H, and C₁-C₆ alkyl.

In another preferred embodiment, R₁, R₂, R₃, R₄, R₅, R₈, R₉, R₁₀, R₆' and R₇' are all H.

In another preferred embodiment, the compound is selected from the group consisting of:

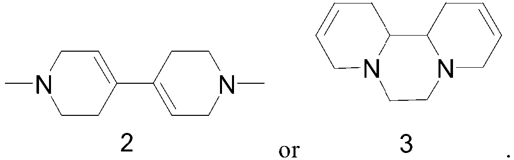

In the second aspect of the present invention, provided is a method for preparing a compound of formula I or II, comprising steps of: 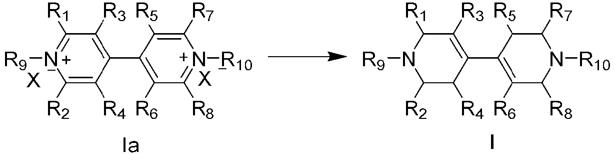 or 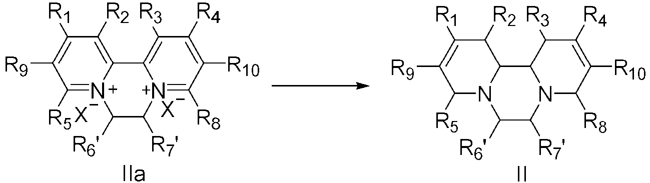
in an aqueous solution, reacting a compound of formula Ia or a compound of formula IIa with a reducing agent to obtain a compound of formula I or a compound of formula II; wherein, the reducing agent is borohydride or H₂;
wherein, X is selected from Cl and Br.

In another preferred embodiment, when the reducing agent is H₂, the reaction is carried out in the presence of a hydrogenation catalyst; preferably, the hydrogenation catalyst is selected from the group consisting of: Pd and Ni.

In another preferred embodiment, the borohydride is selected from the group consisting of: potassium borohydride, sodium borohydride, or a combination thereof.

In another preferred embodiment, in the method, the molar ratio of the compound of formula Ia or the compound of formula IIa to borohydride is 1: (2-5), preferably is 1: (3-4).

In another preferred embodiment, the reaction temperature is 10-40 °C; preferably is 15-30 °C; and/or

In another preferred embodiment, the reaction time is 10-30 minutes; preferably 15-20 minutes.

In another preferred embodiment, the compound of formula Ia is 1,1-dimethyl-4,4 '-bipyridinium dichloride.

In another preferred embodiment, the compound of formula IIa is 1,1 '- ethylene-2,2' - bipyridinium dibromide.

In another preferred embodiment, the method further comprises a step of: separating and purifying the reaction product after the reaction is completed.

In the third aspect of the invention, provided is an agricultural composition, comprising:
(a) the compound of formula I or formula II as described in the first aspect of the present invention, pesticide science acceptable salts, enantiomers, diastereomers, optical isomers, tautomers, racemates, or deuterated derivatives thereof, or combinations thereof, as an active ingredient; and
(b) optional oxidation additives; preferably, the oxidation additive is selected from the group consisting of: oxidant (preferably tetrachlorobenzoquinone, chloramine T, hydrogen peroxide, bleaching powder, sodium dithionite, potassium monopersulfate composite salt), metal catalyst (preferably copper, iron, nickel, molybdenum, ruthenium, manganese, palladium or platinum metal), or a combination thereof; and
(c) pesticide science acceptable carriers and/or excipients; wherein, the preferred carrier is selected from the group consisting of: water, and NaCl aqueous solution; and
(d) optional additives; wherein the additive is selected from the group consisting of: molecular sieve, surfactant, protective colloid, adhesive, thickener, thixotropic agent, penetrant, chelating agent, dye, colourant, polymer, or a combination thereof.

In another preferred embodiment, the content of active ingredient in the agricultural composition is 0.01 - 99.9wt%.

In another preferred embodiment, the oxidation additive is [{Cu(Sal)₂(MeCN)}₂].

In the fourth aspect of the invention, provided is a weeding method, comprising steps of: under the presence of light and oxygen, applying a compound of formula I or II, or an agricultural composition according to the third aspect of the present invention, to the surface of grass or plants that need to be removed, or to the soil or environment around them: 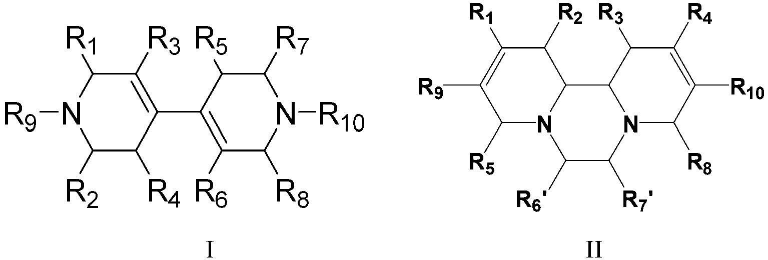 wherein,
R₁, R₂, R₃, R₄, R₅, R₆, R₇, R₈, R₉, and R₁₀ are each independently selected from the group consisting of: hydrogen, halogen, cyano, C₁-C₆ alkyl, C₃-C₆ cycloalkyl, C₂-C₆ alkenyl, C₂-C₆ alkynyl, C₁-C₆ alkoxy, -C(O)O(C₁- C₆) alkyl, -S(O)ₚ(C₁- C₆) alkyl, C₁- C₆ haloalkyl, C₁- C₆ haloalkoxy, and -NRaRb;
p is 0, 1, or 2;
Ra and Rb are each independently selected from the group consisting of: H, C₁-C₆ alkyl, C₃-C₆ cycloalkyl, C₂-C₆ alkenyl, C₂-C₆ alkynyl, C₁-C₆ haloalkyl and C₁-C₆ alkoxy;
R₆' and R₇' are each independently selected from the group consisting of H, C₁-C₆ alkyl, and C₁-C₆ haloalkyl.

In the fifth aspect of the present invention, provided is a compound represented by formula II: 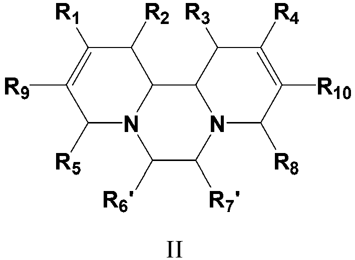 wherein,
R₁, R₂, R₃, R₄, R₅, R₈, R₉, and R₁₀ are each independently selected from the group consisting of: hydrogen, halogen, cyano, C₁-C₆ alkyl, C₃-C₆ cycloalkyl, C₂-C₆ alkenyl, C₂-C₆ alkynyl, C₁-C₆ alkoxy, -C(O)O(C₁- C₆) alkyl, -S(O)ₚ(C₁- C₆) alkyl, C₁- C₆ haloalkyl, C₁- C₆ haloalkoxy, and -NRaRb;
p is 0, 1, or 2;
Ra and Rb are each independently selected from the group consisting of: H, C₁-C₆ alkyl, C₃-C₆ cycloalkyl, C₂-C₆ alkenyl, C₂-C₆ alkynyl, C₁-C₆ haloalkyl and C₁-C₆ alkoxy;
R₆' and R₇' are each independently selected from the group consisting of: H, C₁-C₆ alkyl, and C₁-C₆ haloalkyl;
preferably, the compound is

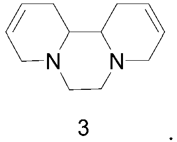

In the fifth aspect of the present invention, provided is a method for preparing paraquat or diquat, comprising the steps of:
under the presence of light and oxygen, the compound of formula 2 or formula 3 undergoes a reaction to obtain paraquat or diquat;

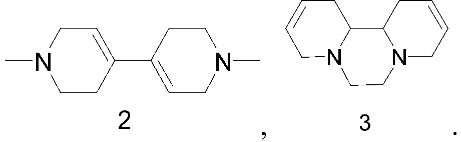

In another preferred embodiment, comprising steps of:
at 60-100 °C, in the presence of an oxidant and catalyst, in the first solvent, the compound of formula 2 or formula 3 undergoes a reaction to obtain paraquat or diquat.

In another preferred embodiment, the oxidant is selected from the group consisting of: tetrachlorobenzoquinone, chloramine T, hydrogen peroxide, bleaching powder, sodium dithionite, potassium monopersulfate composite salt, oxygen, air, or combinations thereof.

In another preferred embodiment, the oxidant is tetrachlorobenzoquinone.

In another preferred embodiment, the catalyst is selected from the group consisting of: copper, iron, nickel, molybdenum, ruthenium, manganese, palladium, platinum, or combinations thereof.

In another preferred embodiment, the catalyst is selected from the group consisting of: [{Cu(Sal)₂(MeCN)}₂], metal chlorides, metal bromides, or combinations thereof; preferably, [{Cu(Sal)₂(MeCN)}₂].

In another preferred embodiment, the metal chloride is selected from the group consisting of: iron chloride, copper chloride, or a combination thereof.

In another preferred embodiment, the metal bromide is selected from the group consisting of: iron bromide, copper bromide, or a combination thereof.

In another preferred embodiment, the amount of oxidant used is 0.2-0.6 times the molar amount of the compound of formula 2 or formula 3

In another preferred embodiment, the amount of catalyst used is 0.01-0.1 times the molar amount of the compound of formula 2 or formula 3.

In another preferred embodiment, the first solvent is selected from the group consisting of: water, C₂-C₆ nitrile solvents, C₁-C₆ ketone solvents, or combinations thereof.

In another preferred embodiment, the first solvent is selected from the group consisting of: water, MeCN, acetone, or a combination thereof.

It should be understood that within the scope of the present invention, the above technical features of the present invention and the technical features specifically described in the following (e.g., embodiments) can be combined with each other, thereby forming a new or preferred technical solution. Due to space limitations, it will not be repeated herein.

### DESCRIPTION OF THE DRAWINGS

Figure 1 shows the trend profile of quantitative analysis and cyclic voltammetry detection of the conversion of the diene-diamine compound to PQ under light and air conditions.
Figure 2 shows the herbicidal performance of the diene-diamine compound on bermuda grass.
Figure 3 shows the herbicidal performance of the diene-diamine compound on acorus calamus.
Figure 4 shows the herbicidal performance of the diene-diamine compound on Arabidopsis thaliana.
Figure 5 shows the herbicidal performance of diquat and its reduced form tricyclodienepiperazine.
Figure 6 shows the effects of intraperitoneal injection of the diene-diamine compound and paraquat (PQ) on the survival rate and body weight of mice, HR: hazard ratio. ***P<0.001, **** P<0.0001, compared to the control group.
Figure 7 shows the data of various biochemical indicators after intraperitoneal injection of the diene-diamine compound and PQ for 48 hours in mice, including TNF - α: tumor necrosis factor alpha; IL-1β: interleukin-1β; IL-6: interleukin-6; SCr: Blood creatinine; BUN: urea nitrogen; UA: Uric acid; AST: Glutamic oxaloacetic transaminase; ALT: Glutamic-pyruvic transaminase; SOD: Superoxide Dismutase; CAT: Peroxidase. P<0.05 is considered statistically significant, with 10 mice per group (n=10).
Figure 8 shows the damage characteristics of major organs in mice after intraperitoneal injection of the diene-diamine compound and PQ for 48 hours. The data is expressed as mean ± standard error, with 6 mice per group (n=6), and P<0.05 indicates statistically significant differences. Scale bar: 50 µm.
Figure 9 shows the transmission electron microscopy results of the main organs in mice after intraperitoneal injection of the diene-diamine compound and PQ for 48 hours.
Figure 10 shows the effects of the diene-diamine compound and PQ on cellular activity and ROS levels in A549 cells, COS-7 cells, and Hep G2 cells.
Figure 11 shows the apoptosis of A549 cells, COS-7 cells, and Hep G2 cells after direct contact with the diene-diamine compound and PQ.
Figure 12 shows the molecular mechanisms of herbicidal activity by the diene-diamine compound and PQ on Arabidopsis thaliana.

### EMBODIMENTS FOR CARRYING OUT THE INVENTION

After extensive and in-depth research, the inventor discovered a non-toxic, practical, cost-effective, and high-performance diene diamine herbicide having a structure of formula I or tricyclic diene piperazine herbicide having a structure of formula II. The herbicide of the present invention does not have the function of an electron transfer agent, and its toxicity is significantly lower than that of paraquat or diquat in the systematic evaluation of in vivo and in vitro experiments, and is comparable to physiological saline which is used as a normal control group. The herbicide of the present invention can effectively convert into active ingredients under natural sunlight and air conditions, achieving efficient weed control performance. Based on this, the inventors completed the present invention.

### Term

Each alkyl, either individually or as part of a larger group (such as alkoxy, alkylthio, alkoxycarbonyl, alkylcarbonyl, alkylaminocarbonyl, or dialkylaminocarbonyl, etc.), can be linear or branched. Typically, the alkyl is, for example, methyl, ethyl, n-propyl, isopropyl, n-butyl, tert butyl, isobutyl, tert butyl, n-pentyl, neopentyl, or n-hexyl.

The term "alkyl" usually refers to C₁-C₆ alkyl, which refers to a straight or branched alkyl group having 1-6 carbon atoms, such as methyl, ethyl, propyl, isopropyl, butyl, isobutyl, sec butyl, tert butyl, or similar groups, preferably is C₁-C₄ alkyl or C₁-C₃ alkyl; more preferably, is C₁-C₂ alkyl (such as methyl).

The alkenyl and alkynyl moieties can be in the form of straight or branched chains, and these alkenyl moiety can have (E) - or (Z) - configurations. The alkenyl and alkynyl moieties can include one or more double and/or triple bonds in any combination; however, it is preferred to include only one double bond (for alkenyl) or only one triple bond (for alkynyl).

The term "C₂-C₆ alkenyl" refers to a straight or branched chain alkenyl group with 2-6 carbon atoms, such as vinyl, allyl, 1-propenyl, isopropenyl, 1-butenyl, 2-butenyl, or similar groups.

The term "C₂-C₆ alkynyl" refers to a straight or branched alkynyl group with 2-6 carbon atoms, such as ethynyl, propynyl, or similar groups.

Typically, the alkenyl or alkynyl group is a C₂-C₄ alkenyl or C₂-C₄ alkynyl group, more specifically ethenyl (vinyl), prop-2-enyl, prop-3-enyl (allyl), ethynyl, prop-3-ynyl (propargyl), or prop-1-ynyl. Preferably, the term "cycloalkyl" refers to cyclopropyl, cyclobutyl, cyclopentyl, or cyclohexyl.

As used herein, the term "aryl" preferably refers to phenyl.

The terms "heteroaryl" and "heteroaromatic ring" (alone or used as part of a larger group (such as heteroaryl-alkyl-)) refer to ring systems containing at least one heteroatom and can be in the form of a monocyclic or bicyclic ring. Preferably, the monocyclic ring will contain 1, 2, or 3 heteroatoms independently selected from nitrogen, oxygen, and sulfur. Typically, as used herein, the term "heteroaryl" includes furyl, thienyl, pyrrolyl, pyrazolyl, imidazolyl, triazolyl, oxazolyl, isoxazolyl, thiazolyl, isothiazolyl, oxadiazolyl, pyridinyl, pyrimidinyl, pyridazinyl, pyrazinyl, and triazinyl rings, which can be substituted or unsubstituted as described herein.

The term "halogen" refers to fluorine, chlorine, bromine, or iodine. The term "halogenated" refers to a group substituted with one or more of the same or different above-mentioned halogen atoms, such as trifluoromethyl, pentafluoroethyl, heptafluoroisopropyl, or similar groups.

Similarly, it applies to halogens in other defined contexts, such as halogenated alkyl or halogenated phenyl.

The halogenated alkyl groups with a chain length of 1 to 6 carbon atoms are, for example, fluoromethyl, difluoromethyl, trifluoromethyl, chloromethyl, dichloromethyl, trichloromethyl, 2,2,2-trifluoroethyl, 2-fluoroethyl, 2-chloroethyl, pentafluoroethyl, 1,1-difluoro-2,2,2-trichloroethyl, 2,2,3,3-tetrafluoroethyl and 2,2,2-trichloroethyl, heptafluoro n-propyl, and perfluoro n-hexyl.

The term "alkoxy" preferably has 1 to 6 carbon atoms. Typically, the alkoxyl is, for example, methoxy, ethoxy, propoxy, isopropoxy, n-butoxy, isobutoxy, sec-butoxy or tert-butoxy, or pentoxy or hexoxy isomer, preferably methoxy and ethoxy. It should be understood that two alkoxy substituents can exist on the same carbon atom.

Typically, the term 'haloalkoxy' refers to, for example, fluoromethoxy, difluoromethoxy, trifluoromethoxy, 2,2,2-trifluoroethoxy, 1,1,2,2-tetrafluoroethoxy, 2-fluoroethoxy, 2-chloroethoxy, 2,2-difluoroethoxy or 2,2,2-trichloroethoxy, preferably difluoromethoxy, 2-chloroethoxy or trifluoromethoxy.

Exemplary "C₁-C₆ alkyl-S- (alkylthio)" includes methylthio, ethylthio, propylthio, isopropylthio, n-butylthio, isobutylthio, sec-butylthio, or tert-butylthio, preferably methylthio or ethylthio.

Exemplary "C₁-C₆ alkyl-S(O)- (alkylsulfinyl)" includes methylsulfinyl, ethylsulfinyl, propylsulfinyl, isopropylsulfinyl, n-butyl sulfinyl, isobutyl sulfinyl, sec-butyl sulfinyl, or tert butyl sulfinyl, preferably methylsulfinyl or ethylsulfinyl.

Exemplary "C₁-C₆ alkyl-S(O)₂- (alkylsulfonyl)" includes methylsulfonyl, ethylsulfonyl, propylsulfonyl, isopropylsulfonyl, n-butyl sulfonyl, isobutyl sulfonyl, sec butyl sulfonyl, or tert butyl sulfonyl, preferably methylsulfonyl or ethylsulfonyl.

The compounds of the present invention may contain one or more asymmetric centers and therefore appear in the form of racemates, racemic mixtures, single enantiomers, diastereomeric compounds, and single diastereomers. The asymmetric centers that can exist depend on the properties of various substituents on the molecule. Each asymmetric center will independently generate two enantiomers, and all possible mixtures of enantiomers and diastereomers, as well as pure or partially pure compounds, are within the scope of the present invention. The present invention includes all isomeric forms of compounds.

The term "under light conditions" refers to the presence of visible light irradiation in the environment, preferably under natural light conditions such as sunlight exposure.

The term "under the presence of oxygen" refers to the presence of sufficient oxygen in the environment to convert the herbicide composition of the present invention into the corresponding active ingredient (paraquat or diquat). In a preferred embodiment, the presence of oxygen may be in an air environment.

The term "pesticide science acceptable salt" refers to the anion of the salt that is known and acceptable when forming a pharmaceutically acceptable salt of the nematode killing agent. Preferably, the salt is water-soluble. Suitable acid addition salts formed from compounds of formula (I) include salts formed from inorganic acids, such as hydrochloride, phosphate, sulfate, nitrate; and salts formed from organic acids, such as acetates, benzoates, etc.

The term "weed" includes unwanted crop species such as volunteer crops, including conventional volunteer crops and volunteer crops genetically modified through mutation or genetic modification methods. For example, in the environment of lawn grasses such as golf courses, if creeping bent grasses were found in a flat lane area where different varieties of grass are cultivated, it can be considered "volunteer". Similarly, when the grasses listed below are found in inappropriate locations, they can be considered weeds.

As used herein, the term "room temperature" refers to 5-45 °C, preferably 10-30 °C; more preferably 25 ± 2 °C.

Unless otherwise specified, in this article, when referring to paraquat (or PQ), it refers to 1,1'-dimethyl-4,4'-bipyridiniumor, or salts thereof formed with halogen anions.

### Non-bipyridyl salt herbicide

The term "active ingredient" or "active substance" or "active compound" refers to a diene diamine compound with a structure of formula I or a tricyclic diene piperazine compound with a structure of formula II, its pesticide science acceptable salts, enantiomers, diastereomers, optical isomers, tautomers, racemates, deuterated derivatives, or combinations thereof; 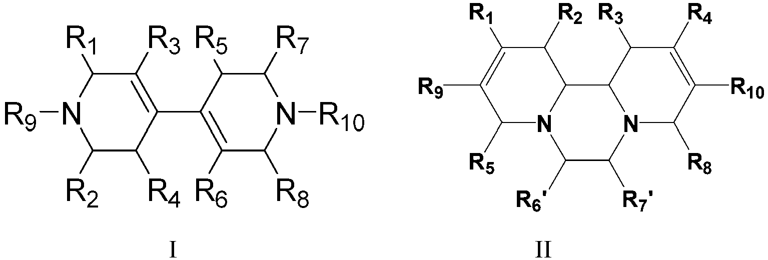 wherein,
R₁, R₂, R₃, R₄, R₅, R₆, R₇, R₈, R₉, and R₁₀ are each independently selected from the group consisting of: hydrogen, halogen, cyano, C₁-C₆ alkyl, C₃-C₆ cycloalkyl, C₂-C₆ alkenyl, C₂-C₆ alkynyl, C₁-C₆ alkoxy, -C(O)O(C₁- C₆) alkyl, -S(O)ₚ(C₁- C₆) alkyl, C₁- C₆ haloalkyl, C₁- C₆ haloalkoxy, and -NRaRb;
p is 0, 1, or 2;
Ra and Rb are each independently selected from the group consisting of: H, C₁-C₆ alkyl, C₃-C₆ cycloalkyl, C₂-C₆ alkenyl, C₂-C₆ alkynyl, C₁-C₆ haloalkyl and C₁-C₆ alkoxy;
R₆' and R₇' are each independently selected from the group consisting of H, C₁-C₆ alkyl, and C₁-C₆ haloalkyl.

The diene diamine compound or tricyclic diene piperazine compound of the present application has herbicidal activity equivalent to paraquat or diquat, but its toxicity to animal bodies is significantly lower than that of PQ or diquat. Therefore, it has great potential for widespread application in green agriculture worldwide and can prevent deaths caused by PQ or diquat.

Examples of weeds that can be removed by the diene diamine compound and the tricyclic diene piperazine structure compound of the present invention include but are not limited to: Bermuda grass, Arabidopsis thaliana, and Acorus calamus. Diene diamine compounds and tricyclic diene piperazine convert to paraquat and diquat under sunlight and air, respectively after contacting the surface of weeds. Both paraquat and diquat are non-selective herbicides that have significant killing effects on weeds and green plants such as poaceae grasses, cyperaceae sedges, broadleaf weeds, asteraceae, polygonaceae, and leguminosae. Diene diamine compounds or tricyclic diene piperazine compounds are mainly used in:
**1)** Weeds in orchard: *Cirsium arvense var. integrifolium, Cirsium japonicum Fisch. ex DC., Conyza japonica, Artemisia lavandulaefolia, Cerastium arvense L., Xanthium strumarium L., Herb of Spanishneedles*)*, Bidens bipinnata), Artemisia hedinii Ostenf. et Pauls, Digitaria sanguinalis (L. ) Scop., Setaria viridis), Eleusine indica (L.) Gaertn., Avena fatua, Vicia hirsuta, Capsella bursa-pastoris, Euphorbia helioscopia.*
**2)** Weeds in paddy fields: *Monochoriavaginalis, Ammannia baccifera, Echinochloa crusgalli*; *Alternanthera philoxeroides, Ophiopogon japonicus, Chrysopogon aciculatus*, *Eleocharis dulcis, Paspalum paspaloides and Sagittaria pygmaea*;
**3)** Weeds in wheat fields: *Aster tataricus L. f*, *Erigeron annuus, Youngia japonica, Erigeron philadelphicus L., Cirsium arvense var. integrifolium, Gnaphalium affine D. Don, Hemistepta lyrata (Bunge) Bunge, Sonchus oleraceus L, Lapsana apogonoides*, *Alopecurus japonicus Steud., Beckmannia syzigachne, Sclerochloa dura, Polypogon fugax Nees ex Steud., Alopecurus aequalis Sobol., Poa pratensis L., Avena fatua*, *Roegneria kamoji, Lolium perenne;*
**4)** Weeds in cotton fields: common ones include *Eleusine indica (L.) Gaertn., Portulaca oleracea L., Digitaria sanguinalis (L. ) Scop., Eclipta prostrata*, *Chenopodium quinoa, Acalypha australis, Sonchus arvensis*, *Echinochloa crusgalli*, *Cyperus rotundus, Imperata cylindrica (L.) Beauv.Bolboschoenus planiculmis*, *Cynodon dactylonlon, Suaeda glauca (Bunge) Bunge, Cirsium arvense var. integrifolium, Amaranthus retroflexus L, Setaria viridis, Solanum nigrum L., Sonchus oleraceus L, Convolvulus arvensis L., Polygonum aviculare L., Parthenocissus tricuspidata (Sieb. & Zucc.) Planch.*, *Commelina diffusa*, *Ipomoea nil (Linnaeus) Roth, Amaranthus viridis, Abutilon theophrasti Medicus, Chloris virgata Sw., Amaranthus roxburghianus, Hibiscus trionum, Xanthium strumarium, Cyperus microiria*, *Daucus carota L., Digitaria ciliaris (Retz.) Koel., Digitaria chrysoblephara*, *Eragrostis pilosa*, *Leptochloa chinensis (L.) Nees, Setaria glauca (L.) Beauv., Cirsium japonicum Fisch. ex DC., Erigeron canadensis, Taraxacum mongolicum Hand.-Mazz., Artemisia scoparia*, *Humulus scandens (Lour.) Merr.,* Physalis minima;
**5)** Weeds in maize field: *Digitaria sanguinalis (L. ) Scop., Leptochloa chinensis (L.) Nees, Portulaca oleracea L., Eleusine indica (L.) Gaertn., etc.;*
6) Weeds in tobacco fields: common weeds include *Acalypha australis, Polygonum lapathifolium L., Echinochloa crusgalli*, *Cardamine hirsuta L., Digitaria sanguinalis (L. ) Scop., Alopecurus aequalis Sobol., Mazus pumilus, etc.;*

Examples of weeds that can be removed by the diene diamine compound and tricyclic diene piperazine herbicide in the presnet invention include but are not limited to: *Cynodon dactylonlon, Arabidopsis thaliana, Acorus calamus L., Digitaria sanguinalis (L.* ) Scop., *Eleusine indica (L.) Gaertn., Eragrostis cilianensis, Portulaca oleracea L., Malachium aquaticum(L.)Fries.*, *Stellaria media (L.) Cyr, Alternanthera Sessilis (Linn.) DC., Cyperus rotundus, Imperata cylindrica (L.) Beauv., Oplismenus compositus (L.) Beauv., Phragmites australis (Cav.) Trin. ex Steud, Galium spurium L., Fallopia convolvulus, Solanum nigrum L., Vicia gigantea Bge., Convolvulus arvensis L., Galeopsis bifida Boenn., Polygonum lapathifolium L., Polygonum bungeanum Turcz, Amaranthus retroflexus L, Commelina communis L., Elsholtzia ciliata (Thunb.) Hyland.*, *ThlaspiarvenseL*, *Vicia sepium L., Descurainia sophia (L.)Webb. ex Prantl, Calystegia hederacea Wall* ) , Avena fatua, *Arrhenatherum elatius (L.) Presl, Aegilops tauschii Coss., Alopecurus aequalis Sobol., Alopecurus japonicas Steud., Aponogeton madagascariensis,* Echinochloa crusgalli, *Poa annua L., Setaria viridis (L.) Beauv., Kyllinga brevifolia Rottb., Cyperus difformis L., Fimbristylis miliacea(L.) Vahl, Juncellus serotinus, Cyperus iria*, Pycreus sanguinolentus, *Bulbostylis barbata (Rotth. ) Kunth, Scirpus juncoides Roxb, Scirpus planiculmis (Fr.) Schmidt, Ajuga nipponensis Makino, Taraxacum mongolicum Hand.-Mazz., Poa pratensis L., Lycopodiastrum casuarinoides, Euphorbia pulcherrima Willd. et Kl., Ananas comosus (Linn.) Merr.,* Polygonum hydropiper L., *Raphanus raphanistrum, Celastrus angulatus Maxim.,* Cardamine hirsuta L., *Echinacea purpurea (Linn. ) Moench,* Inula japonica Thunb and other green weeds .

### Agricultural composition

The active substance of the present invention can be prepared into a weed control composition using conventional methods. These active compounds can be made into conventional preparations, such as solution agents, emulsions, suspensions, powders, foam agents, pastes, granules, aerosols, natural and synthetic materials impregnated with active substances, microcapsules in polymers, coating compound prescription for seeds, and preparations used together with combustion devices, such as smoke cartridge case, smoke cans, and smoke trays, as well as ULV cold mist and warm mist preparations.

These preparations can be produced by known methods, such as mixing active compounds with extenders, which are liquid or liquefied gas or solid diluents or carriers, and surfactants, namely emulsifiers and/or dispersants and/or foam forming agents, can be selected at will. For example, when water is used as an extender, organic solvents can also be used as additives.

If necessary, other active ingredients compatible with the weed control composition of the present invention can be added, such as other herbicides, fungicides, plant growth regulators, antibiotics, insecticides, and fertilizers.

In a preferred embodiment, the content of the active ingredient is 1 to 99.99% by weight based on the total weight of the agricultural composition; preferably, 5-95% by weight, for example, the content of the active ingredient is 10% by weight, 15% by weight, 20% by weight, 25% by weight, 30% by weight, 35% by weight, 40% by weight, 45% by weight, 50% by weight, 55% by weight, 60% by weight, 65% by weight, 70% by weight, 75% by weight, 80% by weight, 85% by weight, 90% by weight, etc.

The pesticide science acceptable carriers and/or excipients include but are not limited to surfactants, protective colloids, adhesives, thickeners, thixotropic agents, penetrants, chelating agents, dyes, colorants, and polymers, and can also be other conventional adjuvants acting as carriers.

The carrier used herein refers to one or more of organic, inorganic, natural products, or synthetic substances. They facilitate the application of active ingredients, and the carrier is generally inert and must be acceptable in agriculture, especially for plants to be treated. The carrier can be solid, such as clay, natural or synthetic silicates, silica, resins, waxes, solid fertilizers, etc; or can be liquid, such as water, alcohols, ketones, petroleum fractions, aromatic hydrocarbons or wax hydrocarbons, chlorinated hydrocarbons, liquefied gases, etc.

When using liquid solvents as diluents or carriers, suitable diluents or carriers may include, for example, aromatic hydrocarbons such as xylene, toluene, or alkyl naphthalene; chlorinated aromatic or chlorinated fatty hydrocarbons, such as chlorobenzene, vinyl chloride, or dichloromethane; Fatty hydrocarbons, such as cyclohexane or paraffin, such as mineral oil fractions; alcohols, such as ethanol or ethylene glycol, as well as their ethers and esters; ketones, such as acetone, methyl ethyl ketone, methyl isobutyl ketone, or cyclohexanone; or uncommon polar solvents such as dimethylformamide and dimethyl sulfoxide, as well as water.

The diluent or carrier of liquefied gas refers to a liquid that will become a gas at normal temperature and pressure, such as aerosol propellants, such as halogenated hydrocarbons, butane, propane, nitrogen, and carbon dioxide.

Solid carriers can be ground natural minerals such as kaolin, clay, talc, quartz, activated clay, montmorillonite, or diatomaceous earth; and ground synthetic minerals, such as highly dispersed silicic acid, alumina, and silicates. The solid carrier used for granules is crushed and graded natural zircon, such as calcite, marble, pumice, sepiolite, and dolomite, as well as particles synthesized from inorganic and organic coarse powders, and particles of organic materials such as sawdust, coconut shells, corn cobs, and tobacco stalks.

Binders, include such as carboxymethyl cellulose and natural and synthetic polymers in the form of powders, granules or lotion, such as gum arabic, polyvinyl alcohol and polyethylene acetate.

The colorants mentioned include for example inorganic dyes such as iron oxide, cobalt oxide, and prussian blue; organic dyes, such as azo dyes or metal phthalocyanine dyes; and trace mineral spplement such as salts of iron, manganese, boron, copper, cobalt, aluminum, and zinc, etc..

The components of the surfactant described in the present invention include emulsifiers, dispersants, or wetting agents, which can be ionic or non-ionic. Examples that can be referred to are: polyacrylates, lignosulfonates, phenolsulfonates or naphthalene sulfonates, polymers of epoxyethane with aliphatic alcohols or with aliphatic acids or with aliphatic amines or with substituted phenols (especially alkylphenols or arylphenols), sulfonic acid succinates, taurine derivatives and alcohol phosphates or polyhydroxyethylated phenol phosphates, alkyl sulfonates, alkyl aryl sulfonates, alkyl sulfates, lauryl ether sulfates, fatty alcohol sulfates, as well as sulfated hexade-heptade-octadecanol and sulfated fatty alcohol ethylene glycol ethers. In addition, there are also condensates of naphthalene or naphthalene sulfonic acid with phenol and formaldehyde, polyoxyethylene octyl benzyl ether, ethoxylated isooctyl ether, octylphenol or nonylphenol, alkyl phenyl polyethylene glycol ether, tributylphenyl polyethylene glycol ether, triethyl phenyl polyethylene glycol ether, alkyl aryl polyether alcohol, condensates of alcohol and fatty alcohol/ethylene oxide, ethoxylated castor oil, polyoxyethylene alkyl ether, ethoxylated polypropylene, lauryl alcohol polyethylene glycol ether acetal, sorbitol ester, lignin sulfite waste liquid, as well as proteins, denatured proteins, polysaccharides, hydrophobically modified starch, polyvinyl alcohol, polycarboxylates, polyoxyalkylates, polyvinylamine, polyvinyl pyrrolidone, and copolymers.

Preferably, the carrier and/or excipient is at least one of emulsifiers, dispersants, wetting agents, spreading agents, stabilizers, defoamers, enhancers, penetrating agents, adhesives, carriers, and fillers.

Preferably, the dosage form of the agricultural composition is selected from at least one of wettable powders, soluble powders, emulsifiers, water suspensions, dispersible oil suspensions, water emulsions, microemulsions, and water dispersible granules.

The present invention imposes no specific limitation on the methods for preparing agricultural compositions herbicides in various formulations. Those skilled in the art can refer to the standard methods provided in "Modern Pesticide Formulation Processing Technology" (edited by Liu Guangwen, Chemical Industry Press) to prepare herbicides with desired formulation specifications.

The components in the herbicide composition provided by the present invention can be stored together or separately. According to a preferred embodiment, each component in the agricultural composition that forms the active ingredient in the herbicide can be stored independently or in a mixture of two or more and mixed in a bucket for immediate use.

The agricultural composition herbicide of the present invention includes, but is not limited to, being used for crops and/or weeds by methods such as spray.

### Preparation methods of diene diamine compounds and tricyclic diene piperazine

The compound with the structure shown in Formula I or Formula II of the present invention can be prepared by the following method, however, the conditions of the method, such as reactants, solvents, bases, the amount of compound used, reaction temperature, reaction time, etc., are not limited to the following explanation. The compounds of the present invention can also be conveniently prepared by combining various synthetic methods described in this specification or known in the art, and such combinations can be easily carried out by those skilled in the art to which the present invention belongs.

Specifically, the compounds of the present invention can be prepared by the following method: 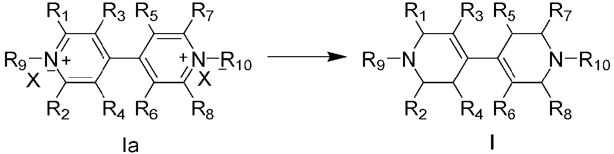 or 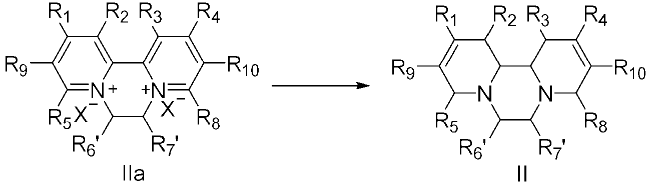
in an aqueous solution, reacting a compound of formula Ia or a compound of formula IIa with a reducing agent to obtain a compound of formula I or a compound of formula II; wherein, the reducing agent is borohydride or H₂;
wherein, X is selected from Cl and Br.

In one embodiment, when the reducing agent is H₂, the reaction is carried out in the presence of a hydrogenation catalyst; preferably, the hydrogenation catalyst is selected from the group consisting of: Pd and Ni.

In another preferred embodiment, the borohydride is selected from the group consisting of: potassium borohydride, sodium borohydride, or a combination thereof. In another embodiment, the molar ratio of the compound of formula Ia or the compound of formula IIa to borohydride in the method is 1: (2-5), preferably 1: (3-4).

In another embodiment, the reaction temperature is 10-40 °C; preferably 15-30 °C; and/or

In another embodiment, the reaction time is 10-30 minutes; preferably 15-20 minutes. wherein,

In another embodiment, the compound of formula Ia is 1,1-dimethyl-4,4 '- bipyridinium dichloride.

In another embodiment, the compound of formula IIa is 1,1 '- ethylene-2,2' - bipyridinium dibromide.

In another embodiment, the method further comprises a step of: separating and purifying the reaction product after the reaction is completed.

### Compared with existing technologies, the main advantages of the present invention include:

(1) The diene diamine compound and tricyclic diene piperazine structure of the present invention have herbicide efficacy equivalent to that of paraquat or diquat, but their toxicity to animal bodies is comparable to that of the control group (physiological saline), the compounds almost have no toxicity. Therefore, they can be used as alternatives to paraquat or diquat.
(2) The diene diamine compound and tricyclic diene piperazine structure of the present invention exhibit herbicidal efficacy under natural light and air conditions.

The present invention was further elaborated hereafter in combination with specific embodiments. It should be understood that these examples are only used to illustrate the invention and not to limit the scope of the invention. The experimental methods without specific conditions in the following examples generally follow the conventional conditions or the conditions suggested by the manufacturer. Unless otherwise stated, percentages and parts are percentages by weight and parts by weight.

### Example 1 Preparation of diene diamine compound 2

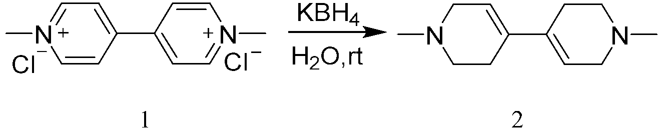

1,1-dimethyl-4,4 '- bipyridinium dichloride (100 g, 0.39 mol, 1.0 equiv.) was dissolved in 400 mL of distilled water, potassium borohydride (98.4 g, 1.17 mol, 3.0 equiv.) was added, and the mixture was stirred at room temperature for 15 minutes and extracted 3 times with dichloromethane (200mL), dried with anhydrous sodium sulfate and evaporated to obtain a white solid (73.5g, yield 96%).¹H NMR (500 MHz, D₂O). δ 5.62 (t, 1H), 2.87 (s, 2H), 2.45 (t, 2H), 2.17 (t, 2H), 2.12 (s, 3H). ¹³C NMR (126 MHz, D₂O) δ 133.5, 119.5, 53.4, 50.6, 43.8, 24.8. HRMS (ESI) m/z calcd for C₁₂H₂₁N₂[(M+H)+]: 193.1626, found: 193.1703.

The above method of the present invention can be used to produce the diene diamine compound on a larger scale, with milder conditions and lower costs compared to existing small-scale production methods.

### Example 2 Preparation of tricyclic diene piperazine (Compound of formula 3)

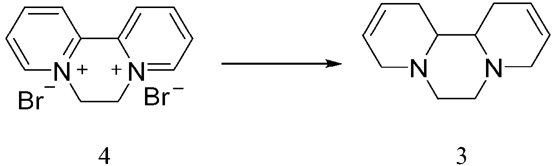

1,1 '- ethylene-2,2' - bipyridinium dibromide (100 g, 0.35 mol, 1.0 equiv.) was dissolved in 400 mL of distilled water, potassium borohydride (56.7 g, 1.05 mol, 3.0 equiv.) was added, and the mixture was stirred at room temperature for 15 minutes and extracted 3 times with dichloromethane (200mL), dried with anhydrous sodium sulfate and evaporated to obtain a black solid (47.1g, yield 70%).¹H NMR (500 MHz, CDCl₃) δ5.6-5.5 (m 4H), 3.1 (m 2H), 2.8-2.7 (m 2H), 2.6 (m 2H), 2.4-2.3 (m 2H), 2.0 (m 2H), 1.9 (m 2H),1.8 (m 2H); ¹³C NMR (126 MHz, CDCl₃) δ 124.0, 123.8, 61.9, 54.0, 53.98, 29.0.

### Example 3 Conversion of Compound of formula 2 under the condition of tetrachlorobenzoquinone

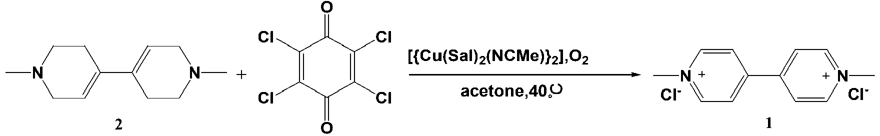

1,1'- dimethyl-1,1', 2,2 ', 3,3', 6,6 '- octahydro-4,4' - bipyridine (100 mg, 0.52 mmol, 1.0 equiv.), tetrachlorobenzoquinone (63.9 mg, 0.26 mmol, 0.5 equiv.), and [{Cu(Sal)₂(NCMe)}₂] (19.7 mg, 0.03 mmol, 0.05 equiv.) were added to 5 mL of acetone and heated to 40 °C to react for 8 hours. The reaction was rinsed twice with 20mL of dichloromethane, and filtered to obtain a black solid; then rinsed twice with 20mL of methanol, dried over sodium sulfate and evaporated to obtain yellow 1,1-dimethyl-4,4 '- bipyridinium dichloride (128mg, 96%).¹H NMR (500 MHz, D₂O) δ 8.91 (d, *J* = 5.7 Hz, 1H), 8.39 (d, *J* = 4.8 Hz, 1H), 4.37 (s, 2H). ¹³C NMR (126 MHz, D₂O) δ 149.8, 146.2, 126.6, 48.3. HRMS (ESI) *m*/*z* calcd for C₁₂H₁₄N₂[(M+2H)/2]: 93.0572, found: 93.0573. The product was verified to be paraquat, indicating that compound of formula 2 can be converted back to paraquat under oxidative conditions.

### Example 4 Conversion of the compound of formula 2 under Natural Conditions

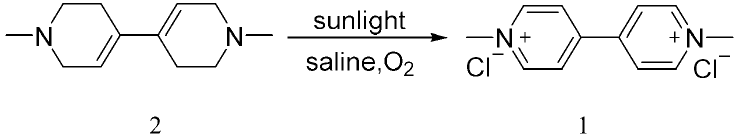

1,1 '- dimethyl-1,1', 2,2', 3,3', 6,6' - octahydro-4,4' - bipyridine (50mg, 0.26mmol, 1.0 equiv.) was added to 5mL of physiological saline and exposed to natural light for 24 hours. A yellow solid (40mg, yield 63%) was obtained. ¹H NMR (500 MHz, D₂O) δ 8.91 (d, *J* = 5.7 Hz, 1H), 8.39 (d, *J* = 4.8 Hz, 1H), 4.37 (s, 2H). ¹³C NMR (126 MHz, D₂O) δ 149.8, 146.2, 126.6, 48.3. HRMS (ESI) *m*/*z* calcd for C₁₂H₁₄N₂[(M+2H)/2]: 93.0572, found: 93.0573. The product was verified to be paraquat.

### Example 5 Conversion of the compound of formula 3 under Natural Conditions

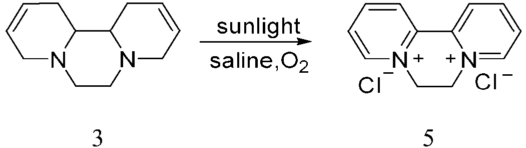

50mg of 1,4,6,7,9,12,12a, 12b octahydrodipyridin[1,2-a:2', 1' - c] pyrazine (50mg, 0.26mmol, 1.0 equiv.) was added to 5mL of physiological saline and exposed to natural light for 24 hours. A yellow solid was obtained. ¹H NMR (500 MHz, D₂O) δ9.1 (dd, 2H), 8.8 (dd, 2H), 8.7 (m, 2H), 8.3-8.2(m, 2H), 5.2(d, 4H). ¹³C NMR (126 MHz, D₂O) δ = 148.26, 147.0, 130.6, 128.3, 52.3. The product was verified to be compound of formula 5.

### Example 6 Conversion of Compound of formula 2

The present invention also carried out quantitative analysis of the conversion of diene diamine compounds to PQ. The present invention provides two conversion methods, both of which can achieve this conversion.

Method I (small scale conversion): Under the action of catalyst and oxidant, the compound of formula 2 can achieve varying degrees of conversion in different solvents under high temperature conditions. Specifically, at 80 °C, with water as the solvent, [{Cu (Sa1)₂(NCMe)}₂] (5%) as the catalyst, and tetrachlorobenzoquinone as the oxidant, the conversion rate can reach up to 92%.

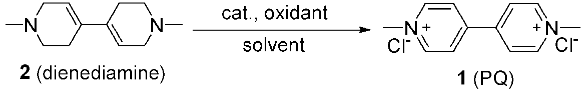

| **Number** | **solvent** | **Cat. (eq)** | **Oxid. (eq)** | **T (°C)** | **Yield (%)** |
|---|---|---|---|---|---|
| 1 | MeCN | FeCl₃(1) | tetrachlorobenzoquinone(1) | 60 | 40 |
| 2 | MeCN | CuCl₂(1) | tetrachlorobenzoquinone(1) | 60 | 55 |
| 3 | MeCN | [{Cu(Sal)₂(NCMe)}₂](1) | tetrachlorobenzoquinone(1) | 60 | 70 |
| 4 | Acetone | [{Cu(Sal)₂(NCMe)}₂](1) | tetrachlorobenzoquinone(1) | 60 | 96 |
| 5 | Acetone | [{Cu(Sal)₂(NCMe)}₂](5%) | tetrachlorobenzoquinone(0.5) | 40 | 96 |
| 6 | H₂O | [{Cu(Sal)₂(NCMe)}₂](5%) | tetrachlorobenzoquinone(0.5) | 40 | 42 |
| 7 | H₂O | [{Cu(Sal)₂(NCMe)}₂](5%) | tetrachlorobenzoquinone(0.5) | 80 | 92 |

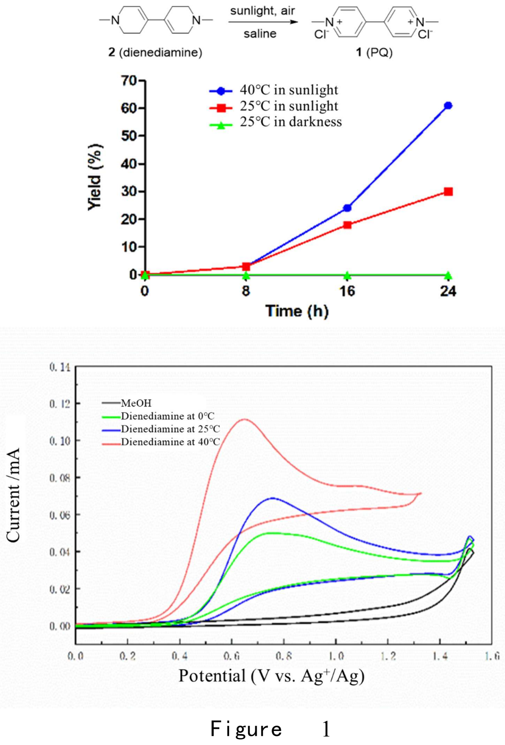

Method II (large scale conversion): Under lighting and air conditions, using physiological saline as the solvent, the compound of formula 2 is converted to PQ. As shown in Figure 1, the compound of formula 2 was placed under dark conditions at 25 °C and lighting conditions at 25 °C and 40 °C, respectively. The results showed that the diene diamine compound hardly converted to PQ under dark conditions, but converted to PQ under lighting conditions, and the conversion efficiency increased with rising temperature. After 24 hours of exposure to natural light at 40 ° C, the conversion rate of the diene diamine compound to PQ was about 63%, and after 24 hours of exposure to natural light at 25 ° C, the conversion rate was about 30%, indicating that the conversion of compound 2 is light dependent and has potential use as a slow-release herbicide.

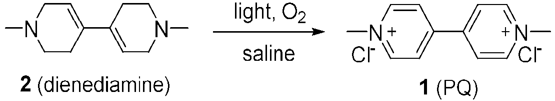

| **Number** | **light source** | **Time (h)** | **T (°C)** | **Yield (%) ^{b}** |
|---|---|---|---|---|
| 1 | darkness | 72 | 25 | 0 |
| 2 | Natural Light | 24 | 25 | 30 |
| 3 | Natural Light | 24 | 40 | 63 |

### Example 7 Evaluation of Herbicidal Performance of the compound of formula 2

As is well-known, Bermuda grass, Arabidopsis thaliana, and Acorus calamus are naturalized green weeds in most parts of the world and are also invasive grasses. Photosynthesis occurs throughout their entire leaves and bundles. The compound of the present invention exhibits good control effects on green weeds by interrupting photosynthesis. Therefore, Bermuda grass, Acorus calamus, and Arabidopsis thaliana were selected as plant models to evaluate the herbicidal performance of the compounds in the present invention.

Under air and sunlight, the compound of formula 2 of the present invention was sprayed on the leaf surface at concentrations of 1, 2, 4, and 8 times the PQ equivalent, respectively. Paraquat herbicide was used as the positive control, and physiological saline was used as the blank control group. The diameter of the observation range was 8cm.

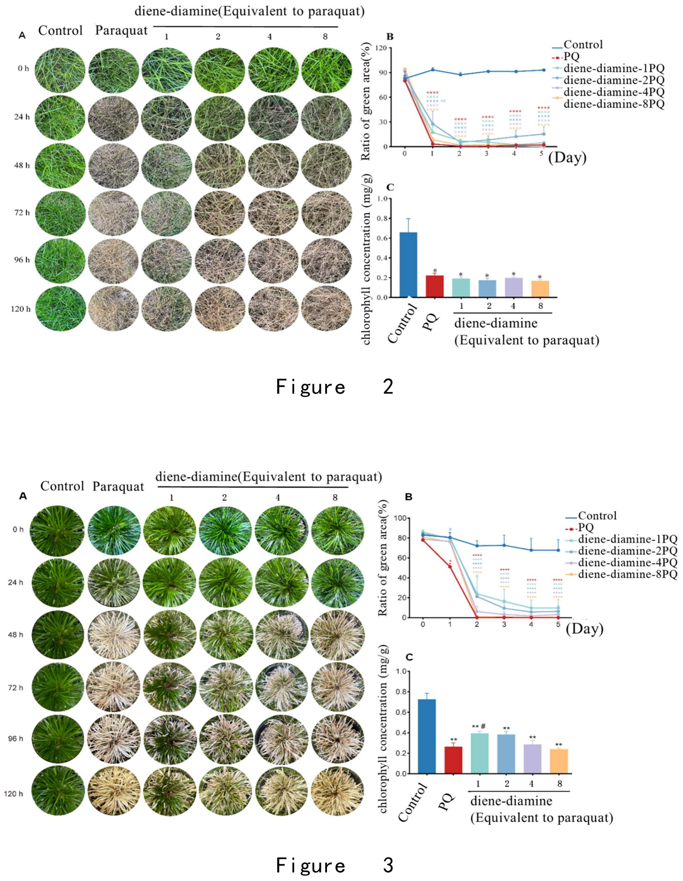

After spraying PQ and diene diamine with various concentrations for 24 hours, the leaves of bermuda grass, acorus calamus, and Arabidopsis thaliana began to wither, as shown in Figures 2, 3, and 4. The weed control effect also increased along with rising concentration. At the same time, it can also be seen that the slightly delayed weed control effect was observed in the groups treated with the same or twice the dose of the compound of formula 2. When the dosage concentration of the compound of formula 2 was greater than twice that of PQ, the herbicidal activity of the compound of formula 2 at any time point after administration was similar to that of PQ. The delayed weed control effect of the compound of formula 2 may be due to its efficiency and yield in converting to PQ under air and sunlight. However, regardless of the dose and concentration, after 120 hours post application, the grass in all groups sprayed with herbicides completely withered and dried. These data demonstrated the herbicidal activity of compound 2, diene diamine.

### Example 8 Evaluation of Herbicidal Performance of the compound of formula 3

Under air and sunlight, the compound of formula 3 of the present invention was sprayed on the surface of leaves, with herbicide diquat as a positive control. The diameter of the observation range was 10cm.

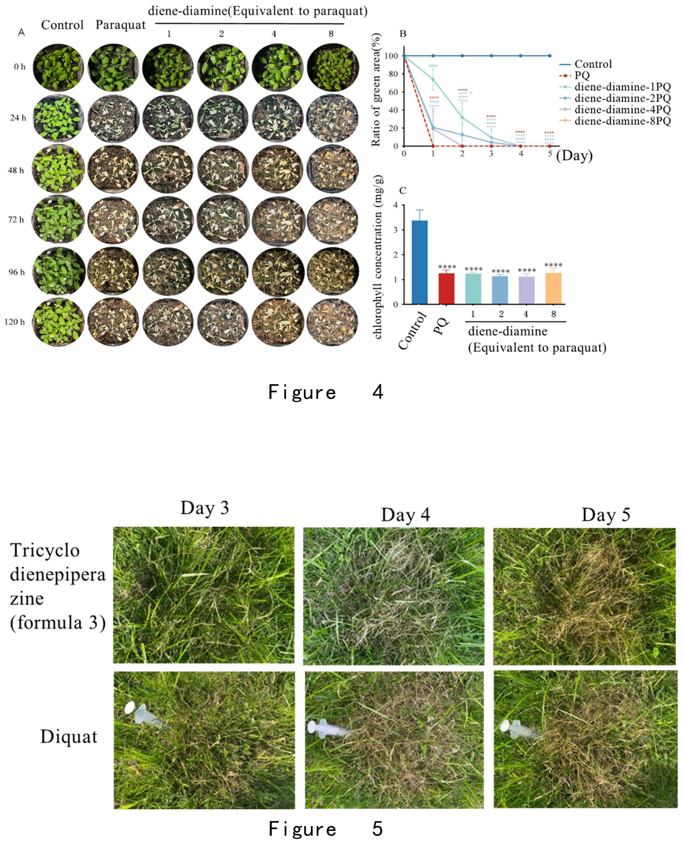

From Figure 5, it can be seen that 3 days after spraying the compound of formula 3 tricyclic diene piperazine (concentration not specified), the leaves of the bermuda grass began to wither. After 5 days, the leaves of bermuda grass completely withered. The compound of formula 3 exhibited a weed control effect comparable to that of diquat, indicating that the compound of the present invention has similar herbicidal performance to diquat.

### Example 9 In vivo safety evaluation of the compound of formula 2

Firstly, the optimal dose and timing for PQ-induced lethality in mice were determined through single intraperitoneal injection at doses of 20, 30, 40, 50, and 60 (mg/kg). All mice survived for over 168 hours at doses of 20 and 30 (mg/kg), but died within 27 to 113 hours at doses of 50 or 60 (mg/kg), with significant weight loss on the first day. In addition, a significant increase in lung injury score was observed 24 hours after PQ administration, with the highest lung injury score observed at 48 hours compared to other time points. Therefore, 50 mg/kg was chosen as the optimal dose. The mouse tissue samples were collected 48 hours after PQ administration.

In order to evaluate the safety of compound 2 of the present invention in vivo, acute toxicity tests were conducted on mice. Firstly, the LD50 of diene diamine in mice was measured. As shown in Table 1, on the third day after administration, all mice survived after intraperitoneal injection of 300 and 600 mg/kg of diene diamine. Three mice died after administration of 900 and 1050 mg/kg, respectively. Eight mice died after administration of 1200 mg/kg. After calculation, the LD50 on the seventh day after diene diamine poisoning was 1003.67 mg/kg (95% CI: 879.34, 111.89). The toxicity of diene diamine in mice is low and the safety is high.

**Table 1 Number of deaths and LD50 determination of acute paraquat poisoning in mice after intraperitoneal injection of paraquat with different concentrations**

| Dosage (mg/kg) | Total number of mice (n) | Mortality, n (%) | |
|---|---|---|---|
| | | Day 1 | Day 7 |
| 300 | 8 | 0 (0) | 0 (0) |
| 600 | 8 | 0 (0) | 0 (0) |
| 900 | 8 | 3 (37.5) | 3 (37.5) |
| 1050 | 8 | 3 (37.5) | 3 (37.5) |
| 1200 | 8 | 8 (100) | 8 (100) |

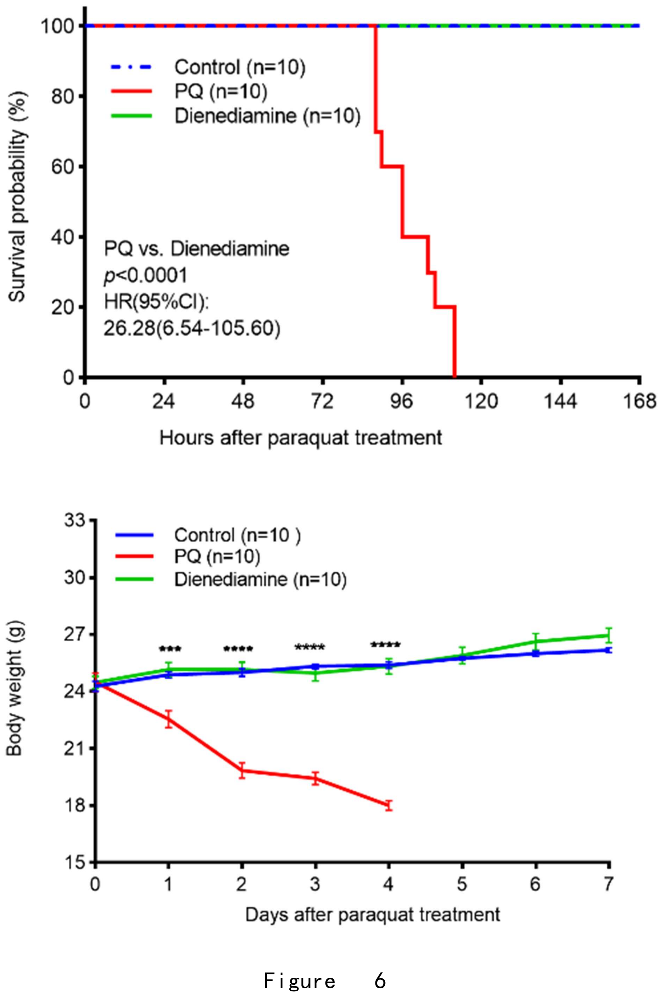

As shown in Figure 6, mice treated with PQ (0.19 mmol/kg) died 100% within 112 hours after PQ administration. In contrast, all mice in **the group of the compound of formula 2** survived after 168 hours of administration of the compound of formula 2(0.19 mmol/kg). The weight of the mice in the PQ group continued to decrease (P<0.01), while in contrast, the weight of the mice in the control group and the **group of the compound of formula 2,** diene diamine group, continued to steadily increase.

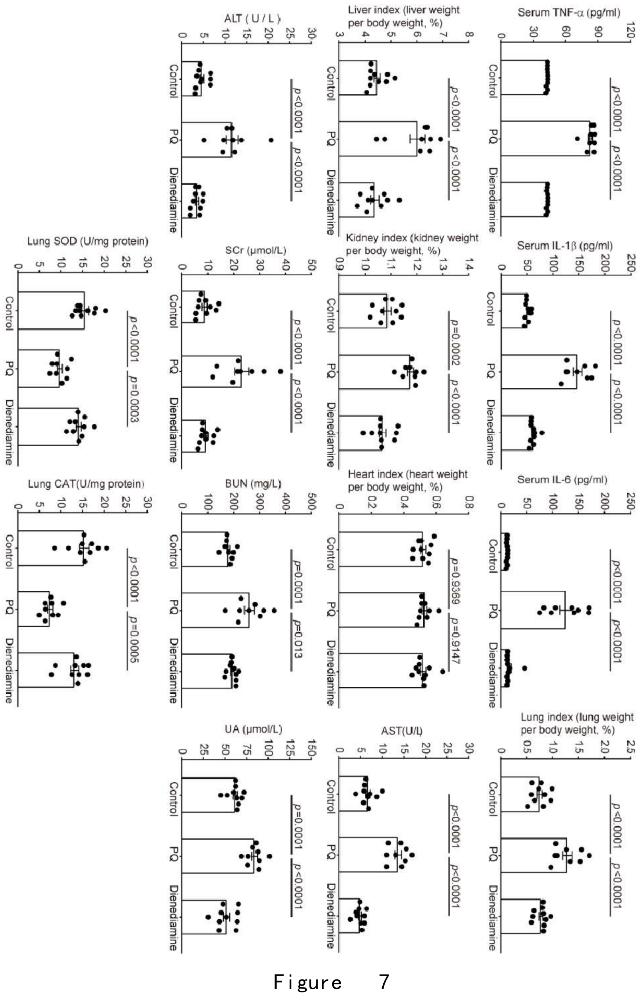

As shown in Figure 7, compared with the control group, the lung, liver, and kidney indices of mice intraperitoneally injected with 50mg/kg paraquat for 48 hours were significantly increased, while there was no statistical difference in lung, liver, and kidney indices between the diene diamine group and the control group. There was no statistical difference in cardiac index among the three groups. The levels of inflammatory markers TNF-α, IL-β, IL-6, liver function AST, ALT, renal function SCr, BUN, and uric acid in the serum of mice in the paraquat group at 48 hour were significantly increased, but the levels of SOD and CAT in lung tissue were significantly decreased (P<0.05). However, in the group of mice injected intraperitoneally with the same dose of the compound of formula 2 diene diamine, there were no significant changes in these indicators. It was indicated that paraquat poisoning at 50mg/kg may lead to systemic inflammation and significant functional impairments in lung, liver, and kidney after 48 hours. However, no significant differences in these biochemical indicators were detected after intraperitoneal injection of the compound of formula 2 diene diamine at the same molar concentration.

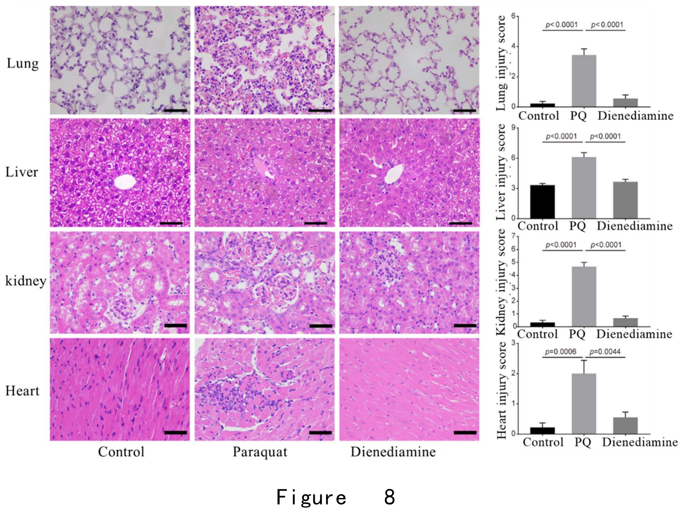

As shown in Figure 8, mice administered with PQ for 48 hours exhibited multiple organic damages accompanied by significant inflammation, including pulmonary hemorrhage, widespread thickening of alveolar septa, hepatic hemorrhage adjacent to central veins, renal interstitial edema, degeneration of renal tubular epithelial cells, and multifocal cardiomyocyte necrosis, accompanied by inflammatory cell infiltration. In contrast, no significant pathological damage was observed in these major tissues in both the diene diamine group and the control group. Meanwhile, the tissue damage scores of the main organs in the PQ group were significantly higher than those in the control group and the diene diamine group.

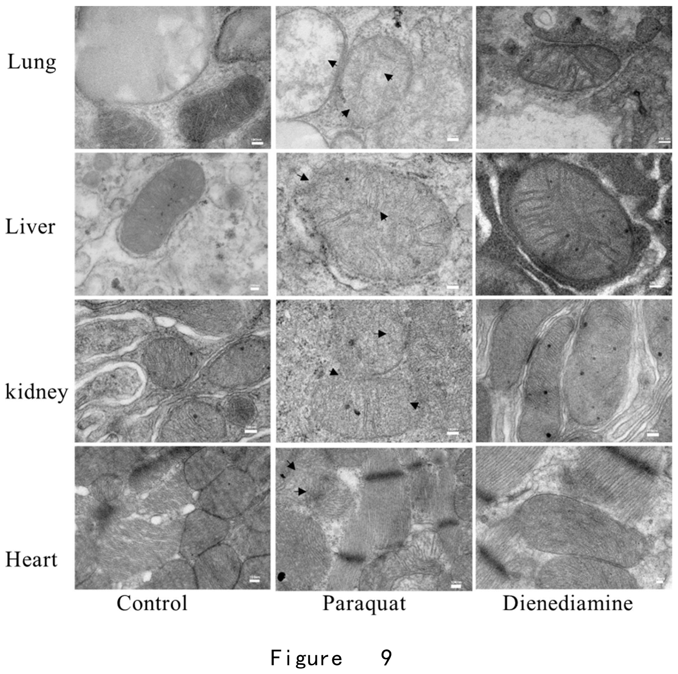

48 hours after intraperitoneal injection of 0.19 mmol/kg of paraquat or the compound of formula 2, transmission electron microscopy samples of the lung, liver, kidney, and heart were collected. The black arrow indicated mitochondrial damage, and the electron microscopy scale of the heart group was 200 nm, and the electron microscope scale of other groups was 100 nm. As shown in Figure 9, transmission electron microscopy results showed mitochondrial damage such as swelling, vacuolization, blurring, or fragmentation in the lung, liver, kidney, and heart tissues of PQ group mice. No significant mitochondrial structural damage was detected in both the diene diamine group and the control group.

PQ is rapidly absorbed and distributed to the lungs, kidneys, liver, and muscles after entering the human body. Death caused by ingestion of PQ is usually due to multiple organ failure, such as pulmonary edema, kidney and liver failure. Therefore, lung cells (A549 cells), kidney cells (COS-7), and liver cells (Hep G2) were selected to evaluate the direct cytotoxicity of diene diamine.

After co-culturing with PQ for 24 hours, PQ exhibited direct cytotoxic effects on A549, Hep G2, and COS-7 cell lines in a dose-dependent manner. In contrast, no significant changes in cell viability were detected under the same concentration gradient after co-incubation with the compound of formula 2 for 24 hours. The half maximal inhibitory concentration (IC₅₀) was calculated to exhibit the cytotoxicity of the compound of formula 2. The results were shown in Table 2:

**Table 2 Toxic effects of diene diamine and paraquat on three types of cells**

| | A549 cells | hep G2 cells | Cos-7 cells |
|---|---|---|---|
| Paraquat PQ | 322.3 µM | 131 µM | 114.4 µM |
| Diene diamine compound of formula 2 | 5770 µM | 1358 µM | 1572 µM |

The results showed that for the above three types of cells, the IC₅₀ value of the group of the compound of formula 2 was significantly higher than that of the PQ treatment group, and its IC₅₀ value increased by dozens of times, indicating a very significant improvement in the safety of the organism with diene diamine.

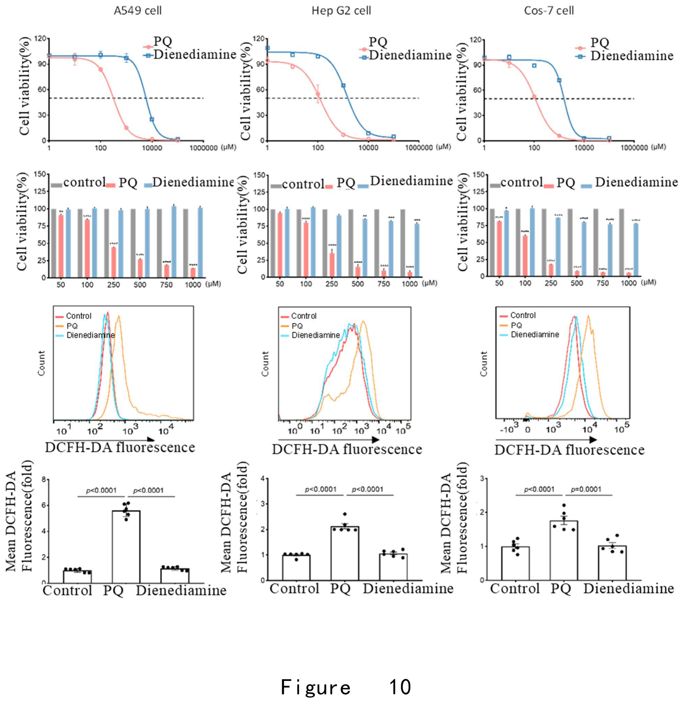

The above three types of cells were exposed to 100 µM PQ (100) and 100 µM of the compound of formula 2 and incubated for 18 hours, and the intracellular reactive oxygen species (ROS) levels were quantified using flow cytometry. Intracellular ROS levels were measured using DCF fluorescence (n=6). As shown in Figure 10, compared with the group of the compound of formula 2 and the control group, the PQ group showed a significant increase in ROS production.

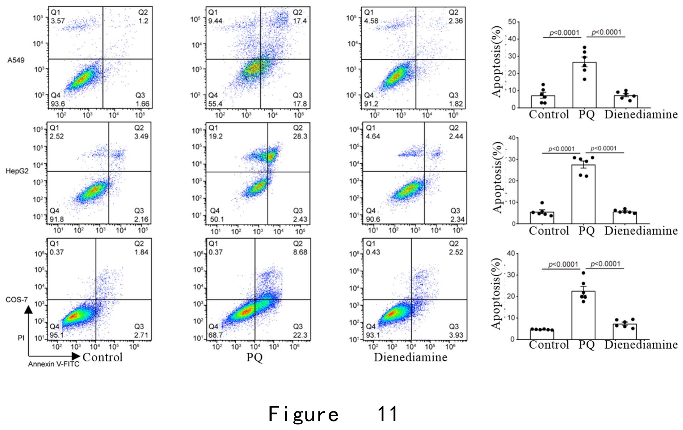

In addition, as shown in Figure 11, after 24 hours of incubation, Annexin V-FITC and propidium iodide (PI) staining (n=6) were performed. The data was expressed as ± SEM. **p* <0.05, *p*<0.05, ***p*<0.01, ****p*<0.001, *****p*<0.0001.

The results showed that compared to the PQ group, the apoptosis rate of the group of the compound of formula 2 was significantly reduced. This result was consistent with ROS levels and there was no significant difference.

### Example 10 Herbicidal mechanism of the compound of formula 2

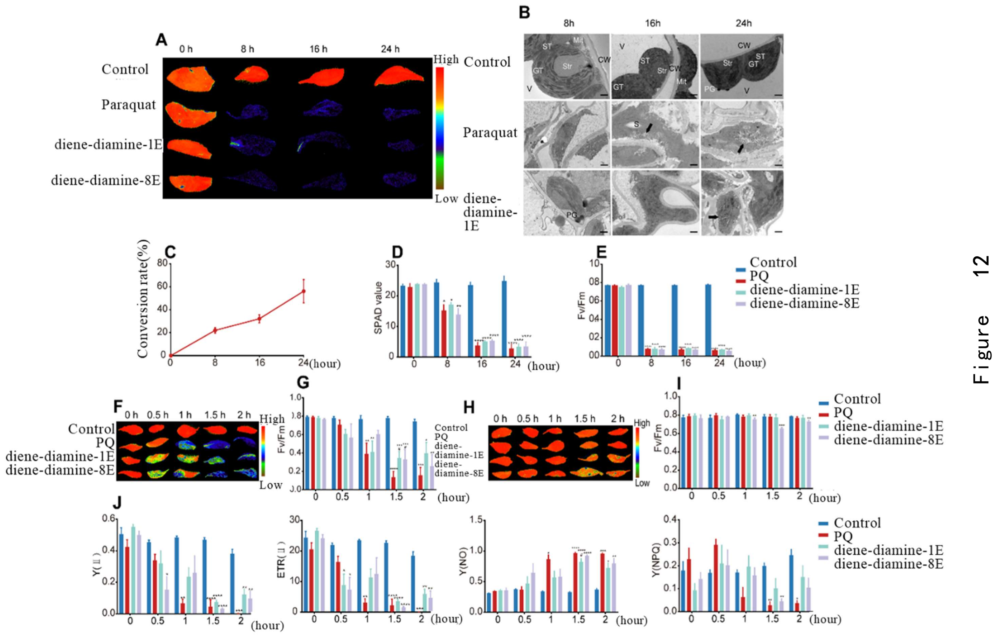

LC-MS detection of paraquat (PQ) in Arabidopsis thaliana sprayed with the compound of formula 2 revealed that after spraying the compound of formula 2 under natural sunlight and air conditions, diene diamine compounds convert to PQ at a time-dependent manner (Figure 12 panel C); Simultaneously, there were significant decreases in chlorophyll content (Figure 4C), maximum fluorescence quantum yield of photosystem II (PSII), maximum photochemical efficiency (Fv/Fm) value, and leaf chlorophyll content index (SPAD) (Figures 12 panel A, panel C, and panel D). Electron microscopy results revealed that spraying the compound of formula 2 resulted in chloroplast stroma lysis, swelling of thylakoids and mitochondrial cristae after 8 hours, progressing to rupture of mesophyll cell nuclei, mitochondria, and chloroplast envelopes after 24 hours, leading to accelerated plant death (Figure 12 panel B). These results demonstrate that the compound of formula 2 sprayed on Arabidopsis thaliana converts to PQ, leading to the withering and death of green plants by interfering with the photosynthetic electron transport mechanism of chloroplasts.

### Brief summary

The present invention discovered for the first time that the compound of formula 2 cannot convert to PQ under air and dark conditions, but can directly convert to PQ in the presence of air and sunlight. In addition, its herbicidal activity is comparable to the herbicidal activity of PQ under natural sunlight and air. All of this evidence suggests that the conversion of the compound of formula 2 to PQ on plants is the key mechanism for its high herbicidal activity.

The present invention has also demonstrated through in vivo and in vitro experiments that the toxicity of the compound of formula 2 to animals is significantly lower than that of PQ, and its toxicity level is comparable to that of the normal control group in which physiological saline is used, indicating that the compound of formula 2, diene diamine, is a non-toxic herbicide.

The bipyridine structure is the basis of PQ as an electron transfer catalyst, which generates a large amount of free radicals and causes cell damage. PQ induces a decrease in SOD and CAT in lung tissue and an increase in ROS in lung, kidney, and liver cells. On the contrary, there were no significant changes in SOD and CAT levels in lung tissue and ROS production in cell lines in the diene diamine group.

In summary, the present invention inventively uses diene diamine 2 to prepare a herbicide composition, which can effectively convert to PQ under natural sunlight and air conditions. Diene diamine compounds demonstrate herbicidal activity comparable to PQ, while exhibiting no toxicity. Therefore, diene diamine compounds have great potential for widespread application in green agriculture worldwide and can prevent deaths caused by PQ.

All documents mentioned in the present invention are cited as references in this application, just as each document is individually cited as a reference. In addition, it should be understood that, after reading the above teaching content of the present invention, those skilled in the art can make various changes or modifications to the present invention, and these equivalent forms also fall within the scope defined by the appended claims of the present application.

## Claims

1. A use of a compound having a structure shown in Formula I or Formula II, a pesticide science acceptable salt, an enantiomer, a diastereomer, an optical isomer, a tautomer, a racemate, a deuterated derivative thereof, or a combination thereof, for the preparation of plant killing agents (preferably herbicides): 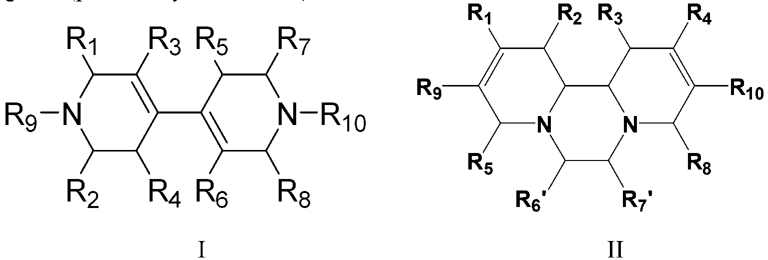 wherein,
R₁, R₂, R₃, R₄, R₅, R₆, R₇, R₈, R₉, and R₁₀ are each independently selected from the group consisting of: hydrogen, halogen, cyano, C₁-C₆ alkyl, C₃-C₆ cycloalkyl, C₂-C₆ alkenyl, C₂-C₆ alkynyl, C₁-C₆ alkoxy, -C(O)O(C₁- C₆) alkyl, -S(O)ₚ(C₁- C₆) alkyl, C₁- C₆ haloalkyl, C₁- C₆ haloalkoxy, and -NRaRb;
p is 0, 1, or 2;
Ra and Rb are each independently selected from the group consisting of: H, C₁-C₆ alkyl, C₃-C₆ cycloalkyl, C₂-C₆ alkenyl, C₂-C₆ alkynyl, C₁-C₆ haloalkyl and C₁-C₆ alkoxy;
R₆' and R₇' are each independently selected from the group consisting of H, C₁-C₆ alkyl, and C₁-C₆ haloalkyl.

2. The use according to claim 1, wherein the compound has a structure shown in formula I, and wherein:
R₁, R₂, R₃, R₄, R₅, R₆, R₇, and R₈ are each independently selected from the group consisting of: hydrogen, halogen, cyano, C₁-C₆ alkyl, C₃-C₆ cycloalkyl, C₂-C₆ alkenyl, C₂-C₆ alkynyl, C₁-C₆ alkoxy, -C(O)O(C₁- C₆) alkyl, -S(O)ₚ(C₁- C₆) alkyl, C₁- C₆ haloalkyl, C₁- C₆ haloalkoxy, and -NRaRb;
R₉ and R₁₀ are each independently selected from the group consisting of: C₁-C₆ alkyl, C₃-C₆ cycloalkyl, C₂-C₆ alkenyl, C₂-C₆ alkynyl, and C₁-C₆ haloalkyl;
Ra and Rb are each independently selected from the group consisting of H, C₁-C₆ alkyl, C₃-C₆ cycloalkyl, C₂-C₆ alkenyl, and C₂-C₆ alkynyl.

3. The use according to claim 2, wherein,
R₁, R₂, R₃, R₄, R₅, R₆, R₇, and R₈ are each independently selected from the group consisting of: hydrogen, halogen, and cyano;
R₉ and R₁₀ are each independently selected from the group consisting of: C₁-C₆ alkyl, and C₃-C₆ cycloalkyl.

4. The use according to claim 2, wherein R₁, R₂, R₃, R₄, R₅, R₆, R₇, and R₈ are all H, and R₉ and R₁₀ are C₁-C₄ alkyl.

5. The use according to claim 1, wherein the compound has a structure shown in formula II, and
R₁, R₂, R₃, R₄, R₅, R₈, R₉, and R₁₀ are each independently selected from the group consisting of: hydrogen, halogen, cyano, C₁-C₆ alkyl, C₃-C₆ cycloalkyl, C₂-C₆ alkenyl, and C₂-C₆ alkynyl;
R₆' and R₇' are each independently selected from the group consisting of: H, and C₁-C₆ alkyl.

6. The use according to claim 5, or a combination thereof, **characterized in that** R₁, R₂, R₃, R₄, R₅, R₈, R₉, R₁₀, R₆', and R₇' are all H.

7. The use according to claim 1, wherein the compound is selected from the group consisting of:

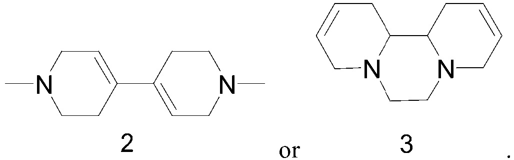

8. A method for preparing a compound of formula I or formula II, wherein comprising steps of: 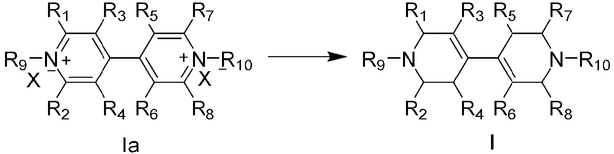 or 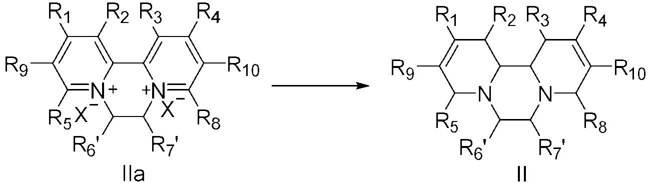
in an aqueous solution, subjecting a compound of formula Ia or a compound of formula IIa to a reaction with a reducing agent to obtain the compound of formula I or a compound of formula II; wherein, the reducing agent is borohydride or H₂;
wherein, X is selected from Cl and Br.

9. An agricultural composition, comprising:
(a) the compound of formula I or formula II according to claim 1, the pesticide science acceptable salt, the enantiomer, the diastereomer, the optical isomer, the tautomer, the racemate, the deuterated derivative thereof, or the combinations thereof, as an active ingredient; and
(b) an optional oxidation additive; preferably, the oxidation additive is selected from the group consisting of: oxidant (preferably tetrachlorobenzoquinone, chloramine T, hydrogen peroxide, bleaching powder, sodium dithionite, potassium monopersulfate composite salt), metal catalyst (preferably copper, iron, nickel, molybdenum, ruthenium, manganese, palladium or platinum metal), or a combination thereof; and
(c) a pesticide science acceptable carrier and/or excipient; wherein, the preferred carrier is selected from the group consisting of: water, and NaCl aqueous solution; and
(d) an optional additive; wherein the additive is selected from the group consisting of: molecular sieve, surfactant, protective colloid, adhesive, thickener, thixotropic agent, penetrant, chelating agent, dye, colourant, polymer, or a combination thereof.

10. A weeding method, wherein comprising steps of: under the presence of light and oxygen, applying a compound of formula I or formula II, or the agricultural composition according to claim 9, to the surface of grass or plants that need to be removed, or to the soil or environment around them: 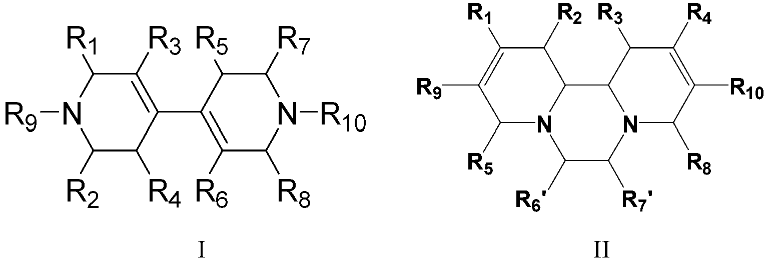 wherein,
R₁, R₂, R₃, R₄, R₅, R₆, R₇, R₈, R₉, and R₁₀ are each independently selected from the group consisting of: hydrogen, halogen, cyano, C₁-C₆ alkyl, C₃-C₆ cycloalkyl, C₂-C₆ alkenyl, C₂-C₆ alkynyl, C₁-C₆ alkoxy, -C(O)O(C₁- C₆) alkyl, -S(O)ₚ(C₁- C₆) alkyl, C₁- C₆ haloalkyl, C₁- C₆ haloalkoxy, and -NRaRb;
p is 0, 1, or 2;
Ra and Rb are each independently selected from the group consisting of: H, C₁-C₆ alkyl, C₃-C₆ cycloalkyl, C₂-C₆ alkenyl, C₂-C₆ alkynyl, C₁-C₆ haloalkyl and C₁-C₆ alkoxy;
R₆' and R₇' are each independently selected from the group consisting of H, C₁-C₆ alkyl, and C₁-C₆ haloalkyl.

11. The compound shown in formula II: 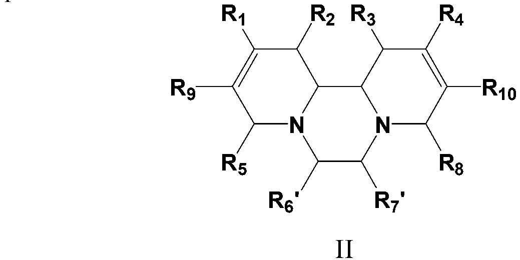 wherein,
R₁, R₂, R₃, R₄, R₅, R₈, R₉, and R₁₀ are each independently selected from the group consisting of: hydrogen, halogen, cyano, C₁-C₆ alkyl, C₃-C₆ cycloalkyl, C₂-C₆ alkenyl, C₂-C₆ alkynyl, C₁-C₆ alkoxy, -C(O)O(C₁- C₆) alkyl, -S(O)ₚ(C₁- C₆) alkyl, C₁- C₆ haloalkyl, C₁- C₆ haloalkoxy, and -NRaRb;
p is 0, 1, or 2;
Ra and Rb are each independently selected from the group consisting of: H, C₁-C₆ alkyl, C₃-C₆ cycloalkyl, C₂-C₆ alkenyl, C₂-C₆ alkynyl, C₁-C₆ haloalkyl and C₁-C₆ alkoxy;
R₆' and R₇' are each independently selected from the group consisting of: H, C₁-C₆ alkyl, and C₁-C₆ haloalkyl;
Preferably, the compound is

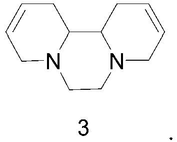

12. A method for preparing paraquat or diquat, wherein comprising the steps of:
under the presence of light and oxygen, the compound shown in formula 2 or formula 3 undergoes a reaction to obtain paraquat or diquat;

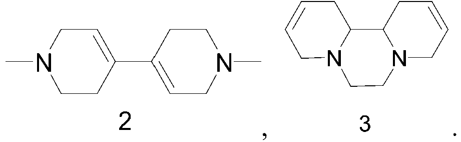

13. The method according to claim 12, wherein comprising steps of:
at 60-100 °C, in the presence of an oxidant and a catalyst, in the first solvent, the compound shown in Formula 2 or Formula 3 undergoes a reaction to obtain paraquat or diquat.
